# EUROPÄISCHE PATENTSCHRIFT

(11) **EP 4 346 649 B1**
(45) Veröffentlichungstag und Bekanntmachung des Hinweises auf die Patenterteilung: **06.08.2025**
(21) Anmeldenummer: 22731506.6
(22) Anmeldetag: 23.05.2022
(51) Int. Cl.: A61B 17/28, A61B 17/16, A61B 17/30, A61B 17/3201

(54) **MEDIZINISCHES INSTRUMENT MIT REINIGUNGSOPTIMIERTER FEDEREINHEIT**
MEDICAL INSTRUMENT HAVING A CLEANING-OPTIMISED SPRING UNIT
INSTRUMENT MÉDICAL DOTÉ D'UNE UNITÉ RESSORT À NETTOYAGE OPTIMISÉ

(30) Priorität: 26.05.2021 DE 102021113559
(43) Veröffentlichungstag der Anmeldung: 10.04.2024
(73) Patentinhaber: Aesculap AG, 78532 Tuttlingen (DE)
(72) Erfinder: DAHMEN, Jan, 78606 Seitingen-Oberflacht (DE)
(74) Vertreter: Winter, Brandl - Partnerschaft mbB
(86) Internationale Anmeldenummer: PCT/EP2022/063927
(87) Internationale Veröffentlichungsnummer: WO 2022/248415

(56) Entgegenhaltungen:
- EP-B1- 2 787 903
- CN-A- 110 785 139
- DE-A1- 10 137 915
- JP-U- H0 531 772
- US-A- 3 921 478

## Beschreibung

### Technisches Gebiet

Die vorliegende Offenbarung betrifft ein medizinisches, insbesondere chirurgisches, Instrument, vorzugsweise ein manuell betätigbares Handinstrument, mit zumindest zwei (einem ersten und einen zweiten) Griffelementen, wobei die Griffelemente insbesondere Metall als Material aufweisen, besonders bevorzugt insgesamt aus Metall als Material hergestellt sind (beispielsweise Edelstahl als Material) und die Griffelemente über ein Lager, insbesondere über ein Gelenk, schwenkbar zueinander gelagert sind, und einer Federeinheit/Federelastische-Baugruppe, welche zwei Feder-Enden aufweist, die jeweils mit einem der beiden Griffelementen verbunden sind, so dass bei Verschwenken zumindest eines der beiden Griffelemente aus einer Grundstellung (entgegen einer elastischen Kraft der Federeinheit) ein Zurückschwenken in die Grundstellung mittels der Federeinheit durchführbar/realisierbar/bewerkstelligbar ist. Daneben betrifft die Offenbarung ein Verfahren zur Herstellung eines medizinischen Instruments.

### Stand der Technik

Bei medizinischen bzw. chirurgischen (Hand-)Instrumenten kommen aktuell Rückstellfedern zum Einsatz, die als Blattfedern ausgebildet sind und an einer Branche bzw. einem Griffelement über einen großen Bereich flächig kontaktierend aufliegen. Sowohl die Blattfeder als auch das Griffelement weisen dabei einander zugewandte großflächige, stetige Oberflächen auf, die sich, ausgehend von der gemeinsamen Kontaktauflagefläche, kontinuierlich voneinander entfernen. Man kann auch sagen, dass die Blattfeder und das Griffelement im Abschnitt der Befestigung eine teilkreis-/bogenförmige Kontur mit unterschiedlichen Radien aufweisen und in Folge sukzessiv einen Abstand zwischen sich (von null an) erhöhen. Während also eine Teiloberfläche der Blattfeder mit einer Teiloberfläche des Griffelements flächig in direktem Kontakt stehen, entfernen sich außerhalb die beiden Oberflächen voneinander und bilden einen sehr kleinen Spalt zwischen sich, der erstallmählich größer wird. In Folge lassen sich die medizinischen Instrumente im Bereich der Kontaktauflagefläche nur sehr schwer reinigen und sterilisieren. Zudem ist die Stelle der Auflage von Rückstellfeder und Griffelement einer Kontaktkorrosion bzw. einer Bimetall-Korrosion ausgesetzt.

Die DE 10 2017 114 260 A1 offenbart beispielsweise ein medizinisches Handinstrument, bei dem eine einteilige Rückstellfeder an einem Griffelement mittels Formschluss angebunden ist. Nachteilig an diesem Instrument ist jedoch, dass aufwendig gefräste Aufnahmen an der Branche bzw. Griffelement bereitgestellt werden müssen. Zudem wird eine einteilige Rückstellfeder vorgesehen, so dass eine linear ansteigende hohe Federkraft bei einem Schließen des Handinstruments entsteht. Diese Federkraft wirkt einer durch einen Nutzer, etwa einem Chirurgen, aufzubringenden Kraft entgegen, insbesondere in einer Schließstellung, und mindert so eine maximal manuell aufbringbare Kraft. Ferner liegt ein hoher Verformungsgrad der Feder vor, so dass eine erhöhte Gefahr eines Brechens der Rückstellfeder im Dauereinsatz besteht. Dokumente JPH0531772U, US3921478A, CN110785139A, DE10137915A1 und EP2787903 offenbaren Geräte des Standes der Technik.

### Zusammenfassung

Die dem Anmeldungsgegenstand zugrundeliegende Aufgabe wird durch ein erfindungsgemäßes Instrument mit den im Anspruch 1 angegebenen Merkmalen, und ein erfindungsgemäße Methode mit den im Anspruch 9 angegebenen Merkmalen, gelöst. Weitere Ausführungsformen sind in den Unteransprüchen angegeben. Es sind die Aufgaben und Ziele der vorliegenden Offenbarung, die Nachteile aus dem Stand der Technik zu vermeiden oder wenigstens zu mindern und insbesondere ein medizinisches, insbesondere chirurgisches, Instrument und ein Verfahren zur Herstellung eines medizinischen Instruments zur Verfügung zu stellen, das eine gute Reinigbarkeit bietet, einfach und kostengünstig in der Herstellung ist und gleichzeitig eine Kraft für ein Verschwenken der Griffelemente über einen großen, insbesondere gesamten, Wegbereich des Instruments konstant hält. Ferner soll das Instrument insbesondere klein aufbauen, ein einfaches Design haben, gut handhabbar sein und eine Wartung vereinfachen.

Die Aufgaben und Ziele hinsichtlich eines medizinisches Instruments werden durch die Merkmale des Anspruchs 1 und hinsichtlich eines Verfahrens zur Herstellung eines medizinischen Instruments durch die Merkmale des Anspruchs 9 gelöst.

Die vorliegende Offenbarung betrifft folglich ein medizinisches Instrument mit zwei relativ zueinander schwenkbaren Griffelementen (Griffbügeln, Griffhebeln, Hebelarmen) und einer Federeinheit, die zwischen den Griffelementen angeordnet ist. Die vorzugsweise U-oder V-förmige Federeinheit weist zwei (Feder-)Enden auf. Jedes der beiden Feder-Enden ist jeweils mit einem entsprechenden/zugehörigen der zwei Griffelemente befestigt, so dass bei Verschwenken zumindest eines der beiden Griffelemente aus einer Grundstellung ein Zurückschwenken in diese Grundstellung realisiert wird. Das bedeutet, wenn eines der beiden Griffelemente von einem Anwender des Instruments manuell verschwenkt wird, kann die Federeinheit das verschwenkte Griffelement wieder in seine Grundstellung zurückschwenken, sobald der Anwender das verschwenkte Griffteil loslässt. Vorzugsweise weist das Instrument gegenüber einem Scharnier, welches die Griffelemente schwenkbar verbindet, Lastarme auf. Das medizinische Instrument kann auch so ausgebildet werden, dass bei der Anwendung des Instruments beide Griffelemente verschwenkt werden. Ist nur ein Griffelement dafür vorgesehen verschwenkt zu werden, dient das andere Griffelement beim Verschwenken nur als Gegenlager für die Hand des Anwenders. Insbesondere weist das medizinische Instrument einen über den proximalen Griffabschnitt betätigbaren distalen Wirkabschnitt wie einen Klemmabschnitt (Klemm-/Fassbranchen) oder Schneidabschnitt (Schneidklingen) etc. auf.

Gemäß der vorliegenden Offenbarung wird also bei dem Instrument eine solche Federeinheit vorgesehen, die sich insbesondere von zumindest einem der beiden Feder-Enden aus, besonders bevorzugt von beiden Feder-Enden aus, hin zu einem Lager der Griffelemente, insbesondere einem Gelenk, besonders bevorzugt einem Scharnier, oder einer Schwenkachse erstreckt und dafür angepasst ist bei Verschwenken der Griffelemente eine konstante/gleichbleibende Kraft/Federkraft/Rückstellkraft bereitzustellen, insbesondere über einen gesamten Schließweg des Instruments (gesamte mögliche Schwenkbewegung). Vorzugsweise wird bei dem Instrument zur Realisierung der gleichbleibenden Federkraft eine zumindest zweiteilige Federeinheit vorgesehen, die zumindest einen ersten Federabschnitt, insbesondere ersten Feder-Schenkel, und einen (separaten) zweiten Federabschnitt, insbesondere zweiten Feder-Schenkel, aufweist, die miteinander verbunden, insbesondere zumindest teilbeweglich miteinander gekoppelt sind. Wenn die beiden Schenkel bzw. Griffelemente von der Grundstellung aus zusammengedrückt werden, kann hierdurch eine Federkraft der Federeinheit (bei Verschwenken in eine Schließstellung), insbesondere über den gesamten Wegbereich des Schließens, gleichmäßig und homogen gehalten werden. Ein linearer Anstieg der Federkraft unterbleibt. Diese spezielle Gestaltung unterstützt einen Nutzer des Instruments und erhöht letztlich auch eine potentiell maximal aufbringbare Schließkraft und wirkt einer Ermüdung des Nutzers entgegen.

Zudem wird die Federeinheit bzw. werden die Feder-Enden noch speziell angepasst mit den Griffelementen verbunden. Konkret ist zumindest ein Feder-Ende formschlüssig in eine zugehörige an dem Griffelement ausgebildete geometrische Aufnahme/Vertiefung eingesteckt und ist stoffschlüssig mit dem zugehörigen Griffelement verbunden. Ein Einstecken des Feder-Endes in die Vertiefung bewirkt, dass das Feder-Ende in zumindest einer Richtung (und insbesondere Gegenrichtung) durch den Formschluss bzw. Hinterschnitt (und ferner insbesondere einer Rotation) gegenüber dem Griffelement geometrisch festgelegt wird. Damit werden zumindest ein, insbesondere zumindest zwei mögliche Bewegungsfreiheitsgrade (von insgesamt sechs Freiheitsgraden: drei translatorische und drei rotatorische) des Feder-Endes gegenüber dem zugehörigen Griffelement gesperrt, insbesondere in eine Richtung der Griffelemente-Längsachse sowie insbesondere eine Rotation um eine Rotationsachse senkrecht auf die Grifflängsachse und senkrecht zur Feder-Enden-Längsachse.

Diese Gestaltung ermöglicht eine einfache und kostengünstige Konstruktion des medizinischen Instruments, welches gut und leicht reinigbar und streilisierbar ist sowie hohen Anforderungen eines Medizintechnikproduktes genügt. Insbesondere ist die Federeinheit nicht verlierbar/verliersicher an den Griffelementen angebunden. Da insbesondere die Federeinheit in allen Schwenkstellungen, insbesondere in der Grundstellung, einzig und allein nur an dem zumindest einen Feder-Ende mit dem zugehörigen Griffelement in Kontakt kommt, ist durch die definierte Verbindungsstelle von Feder-Ende und Griffelement, insbesondere der Aufnahme/Vertiefung, eine individuell angepasste geometrische Gestaltung möglich. Wenn zudem noch das Feder-Ende mit dem Griffelement derart miteinander stoffschlüssig verbunden werden, dass sich eine geschlossene Oberfläche im Verbindungsbereich ausbildet, wird eine gute Reinigung sichergestellt. Bei einer Vertiefung ist zumindest eines der beiden Feder-Enden in eine Vertiefung hineinstehen und mit dem Griffelement stoffschlüssig verbunden sein, dass ein sich ausbildender Spalt zwischen der Vertiefung und dem Feder-Ende zu einer Instrumentenumgebung hin hermetisch verschlossen ist, um einer Keimbildung vorzubeugen.

Mit anderen Worten sind die Federeinheit, insbesondere ein Feder-Schenkel, und zumindest ein Griffelement dafür angepasst, dass ein Feder-Ende in das Griffelement bzw. Branche einsteckbar ist und stoffschlüssig mit diesem verbunden ist, insbesondere derart, dass sich kein Schmutz in der Schnittstelle, etwa in einem Spalt, eindringen oder sich festsetzen kann. Insbesondere ist zumindest einer der beiden Feder-Schenkel in der Branche eingesteckt und ferner derart stoffschlüssig verbunden, dass an der Verbindungsstelle eine hermetisch geschlossene Oberfläche ohne Hinterschnitte vorliegt. Bei einem Einstecken des Feder-Endes in eine zugehörige Vertiefung wird auch einem Ausknicken vorgebeugt.

Die Federeinheit und die Griffelemente können so separat gestaltet und kostengünstig hergestellt werden und werden bei einer Montage sicher miteinander verbunden.

Insbesondere kann auch eine Kontaktstelle/Verbindungsstelle der beiden Feder-Enden insbesondere werkzeuglos trennbar gestaltet sein, um eine leichte und effektive Reinigung oder einen Austausch der Federeinheit sicherzustellen.

Mit noch anderen Worten steht zumindest eines der beiden Feder-Enden in eine in dem zugehörigen Griffelement ausgebildete entsprechende, insbesondere komplementäre Vertiefung (als Aufnahme) (formschlüssig) hinein, die insbesondere zumindest einen Hinterschnitt in distaler Richtung entlang einer Grifflängsachse bildet und ist mit dem zugehörigen Griffelement stoffschlüssig verbunden, insbesondere derart, dass ein Spalt zwischen Vertiefung und Feder-Ende zu einer Instrumentenumgebung hin hermetisch verschlossen ist.

Der Begriff Grundstellung definiert eine Stellung der verschwenkbaren Griffelemente zueinander, in welche sie sich ohne äußere (manuelle) Krafteinwirkung, nur bedingt durch die Federeinheit, begeben. Diese Grundstellung kann insbesondere eine (geometrisch bedingte) Maximalöffnungsstellung des Instruments sein.

Vorzugsweise erstreckt sich also die Federeinheit ausgehend von zumindest einem Feder-Ende hin zu einer Schwenkachse oder einem Lager des medizinischen Instruments, insbesondere nach distal. Insbesondere erstreckt sich zumindest eine Feder ausgehend von ihrem Feder-Ende hin zu der Schwenkachse oder dem Lager, vorzugsweise nach distal. Mit anderen Worten kann die Federeinheit, insbesondere können die zumindest zwei Federn, zwischen den beiden schwenkbaren Griffelementen gelegen entlang dieser Griffelemente hin zu einer Schwenkachse oder zu einem Lager, insbesondere einem Scharnier, verlaufen. Die Federeinheit bzw. die Federn stehen also nicht nach proximal oder von dem Lager abgewandt ab. Hierdurch lässt sich ein Bauraum noch besser nutzen und eine Ergonomie wird weiter verbessert. Der Chirurg muss nicht aufpassen, mit der Federeinheit ungewollt an externen Objekten wie etwa einem OP-Tuch zu verhaken oder auch externe Objekte, beispielsweise ein Gewebe, versehentlich zu beschädigen. Die Federeinheit liegt sozusagen insbesondere zwischen den Griffelementen bzw. ist zwischen den Griffelementen (auf ähnlicher Höhe einer Längsachse des Instruments) angeordnet und ist durch die Griffelemente gewissermaßen umschlossen und schützt durch die Griffelemente die Federeinheit nach (lateral) außen.

Insbesondere kann die Vertiefung innenseitig des zugehörigen Griffelements vorgesehen sein (also gegenüberliegend des anderen Griffelements) und/oder eine Längsachse der Vertiefung kann zu dem gegenüberliegenden Griffelement weisen. Dadurch, dass die Vertiefung nach lateral innen geöffnet ist und in diese das Feder-Ende eingesetzt ist, ist die Federeinheit einerseits verliersicher gehalten und andererseits kann die Federraft besonders gut auf das Griffelement übertragen werden. Die Vertiefung ist also gerade nicht am proximalen Ende des Griffelements ausgebildet, sondern innenseitig des Griffelements. Hierdurch wird in Zusammenwirken mit der Federeinheit insbesondere ein Bauraum und eine Anordnung der Federeinheit optimiert.

Vorteilhafte Ausführungsformen sind in den Unteransprüchen beansprucht und werden insbesondere nachfolgend erläutert.

Gemäß einem Aspekt der vorliegenden Offenbarung kann das zumindest eine Feder-Ende und das zugehörige Griffelement Verschweißt und/oder Verlötet, insbesondere mit einem Zusatz Hartgelötet, und/oder Verklebt sein, insbesondere an einer Kontaktregion/Kontaktgrenze zwischen einer Außenfläche des Feder-Endes und einer innenseitigen Oberfläche des zugehörigen Griffelements, die dem anderen Griffelement gegenüberliegt. Diese stoffschlüssigen Verbindungen sind langlebig, einfach in der Erstellung und stellen insbesondere bei einer umlaufenden Schweißnaht oder Lötnaht eine gegenüber der Umgebung, insbesondere hermetische, Grenze dar, in die keine Keime eindringen können und zudem selbst bei hohen Temperaturen sterilisierbar sind.

Gemäß einem weiteren Aspekt der vorliegenden Offenbarung kann zumindest einer der beiden Feder-Schenkel einen Auskrag-Winkel zwischen dem, insbesondere einstehenden, Feder-Ende und dem zugehörigen Griffelement (distal des Feder-Endes in Branchen-Längsachse gesehen) von mindestens 30°, bevorzugt von mindestens 45°, ganz bevorzugt von mindestens 70° und insbesondere genau 90° aufweist. Man kann auch sagen, der Auskrag-Winkel definiert einen Winkel zwischen einer Griffelementen-Längsachse und einer Feder-Enden-Längsachse. Dieser Mindest-Auskrag-Winkel bewirkt, dass ein Spalt mit ausreichend stumpfen Winkel ausgebildet wird, der sich entsprechend gut reinigen lässt.

Insbesondere beträgt eine Abmessung von einer Stirnseite des Feder-Endes entlang eines geradlinigen Abschnitts des Feder-Endes mindestens 2mm, bevorzugt mindestens 5mm, besonders bevorzugt mindestens 10mm, um eine Mindestspalthöhe zu realisieren. Insbesondere weist ein sich ausbildender Spalt zwischen einem Feder-Schenkel und dem zugehörigen Griffelement eine Mindesthöhe von 1mm, bevorzugt 3mm, besonders bevorzugt 5mm auf. Vorzugsweise beträgt ein Abstand in Richtung senkrecht zur Griffelementlängsachse zwischen einer innenseitigen Oberfläche des Griffs beim Feder-Ende und einer zugewandten Oberfläche der Federeinheit nach einer proximalen Biegung und/oder bei einer Stelle bei einer Länge des Feder-Schenkels von 15% der Gesamtlänge (gemessen von dem Feder-Ende aus), in zumindest der Grundstellung, mindestens 1mm, bevorzugt 3mm und besonders bevorzugt 5mm. So lässt sich das Instrument gut reinigen.

Vorzugsweise kann zumindest ein Feder-Schenkel als Blattfeder oder als Federstahldraht ausgebildet sein und insbesondere als Material den Werkstoff X20CR13 oder nach DIN EN 10088 den Werkstoff 1.4021 aufweisen oder gesamt aus diesem hergestellt sein. Wenn der Feder-Schenkel oder die gesamte Federeinheit aus diesem Werkstoff besteht, wird eine sehr gute mechanische Eigenschaft, eine sehr gute Polierbarkeit, eine gute Schmiedbarkeit gepaart mit guter chemischer Beständigkeit und befriedigender Schweißbarkeit bereitgestellt.

Gemäß einer Ausführungsform kann die Federeinheit zweiteilig in Form von zwei Blattfedern oder zwei Federstahldrähten ausgebildet sein, die über eine distale Feder-Kopplung, insbesondere über eine distale Gabel-Nasen-Verbindung oder eine distale Kugel-Pfannen-Verbindung oder eine distale Y-t-Verbindung miteinander verbunden/gekoppelt sind. Blattfedern sind einfach und kostengünstig in der Herstellung, lassen sich insbesondere einfach irreversibel in die gewollte Form biegen, und bieten zuverlässige Federkräfte. Federstahldrähte auf der anderen Seite sind ebenfalls kostengünstig und erfüllen Anforderungen an eine mechanische Eigenschaft für eine entsprechende Umformung. Insbesondere weisen sie mit ihrem insbesondere kreisrunden Querschnitt keine Kanten auf, so dass sie sich besonders gut reinigen lassen. Bei der zweiteiligen Form mit der distalen Feder-Kopplung bleiben die Federkräfte über den gesamten Schließweg des Instruments bzw. beim Verschwenken der Griffelemente gegeneinander in jeder Schwenkstellung konstant. Ein linearer Anstieg der Federkraft, wie etwa bei einem einteiligen Federelement, wird vermieden.

Gemäß einer weiteren Ausführungsform kann im Falle einer zumindest zweiteiligen Federeinheit zumindest einer der beiden Feder-Schenkel, insbesondere beide Feder-Schenkel, S-förmig ausgebildet sein mit insbesondere einem ersten proximalen Biegeradius und/oder einem zweiten gegenläufigen distalen Biegeradius, wobei vorzugsweise ein Kurven-Winkel/Biege-Winkel zwischen den tangentialen Abschnitten des proximalen Biegeradius (bzw. des Teilkreissegments) 90° und/oder des distalen Biegeradius 45° beträgt. Die S-förmige Gestaltung unterstützt eine federelastische Kraft und geometrische Änderung bzw. Annäherung des Feder-Schenkels gegenüber dem Griffelement, insbesondere derart, dass dieser in keiner Schwenkstellung, zumindest aber nicht in der Grundstellung, der Griffelemente zueinander in Kontakt bzw. Anlage mit dem Griffelement kommt.

Insbesondere kann die Vertiefung ein Sackloch oder eine Nut oder ein Schlitz, insbesondere ein Durchgangsschlitz, sein. In ein Sackloch mit einem definierten Durchmesser kann etwa ein Federstahldraht mit (etwa) gleichem Durchmesser (Presspassung oder Spielpassung; ähnlich eines Stifts in einer Bohrung) eingesteckt werden. Wenn eine Blattfeder als Feder-Schenkel Verwendung findet, so kann das blattförmige/plattenförmige Feder-Ende (mit relativ gesehen geringer Höhe als Breite) in eine langgezogene Nut eingesteckt werden. Auch kann etwa, wenn es sich um eine durchgängige Nut handelt, die Blattfeder auch seitlich in die Nut eingeschoben statt entlang ihrer Längsachse eingesteckt zu werden. Auch kann die Nut einen Hinterschnitt in Einsteckrichtung aufweisen und das Feder-Ende einen zugehörigen Vorsprung, so dass, wenn seitlich eingeschoben, ein Formschluss in Richtung Feder-Enden-Längsachse ausgebildet ist. Zudem kann die Vertiefung insbesondere eine Tiefe (in Richtung einstehender Feder-Enden-Längsachse von mindestens 2mm und/oder maximal 6mm aufweisen. Die Mindesttiefe sichert eine ausreichende mechanische Belastbarkeit des Feder-Schenkels bzw. der Federeinheit

Gemäß einem weiteren Aspekt kann die Federeinheit gegenüber den Griffelementen derart angepasst sein, dass der erste und/oder zweite Feder-Schenkel in zumindest der Grundstellung, insbesondere in allen Stellungen einer Verschwenkung der Griffelemente zueinander, nur an dem Feder-Ende als Verbindungsstelle mit den zugehörigen Griffelementen in Kontakt kommt. So wird ein Abrieb verhindert und eine Haltbarkeit der Federeinheit als auch der Griffelemente verbessert.

Vorzugsweise kann die Federeinheit eine distale Feder-Kopplung aufweisen, die lösbar verschieblich und/oder verschwenkbar zueinander gelagert ist.

Vorzugsweise ist die Vertiefung und/oder das Feder-Ende eckig oder oval (und nicht kreisrund) ausgestaltet, so dass auch ein Rotationsgrad um die Feder-Ende-Längsachse gesperrt ist. Insbesondere weisen Feder-Ende und Vertiefung eine solche Form auf, dass lediglich ein einziger geometrischer Freiheitsgrad in nur einer Richtung verbleibt, der weiter vorzugsweise durch eine Vorspannkraft, etwa bedingt durch die Federeinheit, beaufschlagt ist, um die Federeinheit verliersicher zu halten. Alternativ oder zusätzlich kann das Feder-Ende auch bzw. noch stoffschlüssig mit dem Griffelement verbunden sein.

Vorzugsweise kann das medizinische Instrument als mehrgelenkige Branchenzange ausgeführt sein. Insbesondere kann das medizinische Instrument als mehrfach übersetzte, insbesondere doppelt übersetzte, Zange(n), besonders bevorzugt als doppelt übersetzte Rongeure ausgeführt sein. Für diese Art von Instrument ist der vorliegend offenbarte Federaufbau besonders geeignet. Ein solcher Instrumententyp einer mehrfach bzw. doppelt übersetzten Zange, insbesondere Rongeure, weist mehrere Gelenke auf, die besonders stabil ausgebildet sind. In Folge weisen die Gelenke in Summe jedoch auch eine höhere innere Reibung auf (mehrere Gelenke bedingen eine größere Reibungsoberfläche), welche eine höhere Rückstellkraft der Feder(-einheit) erfordern. Dies stellt einen Unterschied zu Anforderungen an eine Rückstellfeder einer neurologischen Schere oder Zange dar. Bei dem Instrumententyp einer doppelt übersetzen Zange ist es besonders nachteilig, wenn sich die Federkraft im Verlauf des Schließvorgangs erhöht, da die Federkraft bereits zu Beginn des Schließvorgangs sehr hoch ist. Dadurch wird die benötigte, vom Nutzer manuell aufzubringende Handkraft, beim Schließen der doppelt übersetzen Zange zu einer besonders großen Herausforderung, insbesondere, da mit den doppelt übersetzten Rongeuren Knochenstücke abgetrennt werden sollen, wobei diese Tätigkeit an sich bereits eine erhebliche Kraft benötigt. Insbesondere ist es für diese doppelt übersetzten Rongeure vorteilhaft, dass die Vertiefung(en) oder die Aufnahme(n) in dem oder den Griffelement(en) schräg zu einem Lager, insbesondere Gelenk, oder einer Schwenkachse hin verlaufen, da eine solche Konfiguration ein Herausrutschen der Federenden bei großer Federkraft sicher verhindert. Im Gegensatz hierzu sind senkrechte Aufnahmen oder Vertiefungen, also Aufnahmen die sich gegenüber einer Längsachse des zugehörigen Griffelements senkrecht erstrecken, oder solche die gar zu einem Nutzer sich erstrecken, besonders anfällig, bei hoher Kraftaufbringung aus der Vertiefung bzw. Aufnahme herausgedrückt zu werden und das Instrument unbrauchbar zu machen und zudem eine Sicherheit eines Patienten zu gefährden.

Die Aufgaben und Ziele der Offenbarung werden hinsichtlich eines Verfahrens zur Herstellung eines medizinischen Instruments mit einer Federeinheit, insbesondere eines Instruments der vorliegenden Offenbarung, gelöst durch die Schritte, insbesondere in dieser Reihenfolge: Biegen zumindest eines Feder-Schenkels, insbesondere einer Blattfeder oder eines Federstahldrahts; Formen, insbesondere Prägen und/oder Anschleifen, eines distalen Abschnitts des Feder-Schenkels, vorzugsweise ein distales Anschleifen einer Rundung; Einstecken des zumindest einen Feder-Schenkels in eine in einem Griffelement ausgebildete Vertiefung, insbesondere in Form eines Sacklochs oder einer Nut; Vorzugsweise Ausrichten des Feder-Schenkels gegenüber dem zugehörigen Griffelement; Stoffschlüssiges Verbinden, insbesondere Verschweißen und/oder Löten, des Feder-Schenkels mit dem zugehörigen Griffelement. Durch diese Schritte des Verfahrens wird eine Baugruppe eines medizinischen Instruments mit zumindest einem Griffelement und einem Feder-Schenkel bereitgestellt, das einfach und kostengünstig herstellbar ist, sich gut reinigen lässt. Eine Herstellung inklusive simpler Montage ist einfach durchführbar und zudem kostengünstig.

Vorzugsweise kann das Verfahren nach dem Schritt des stoffschlüssigen Verbindens den Schritt Härten des Feder-Schenkels mit dem zugehörigen Griffelement aufweisen und/oder den Schritt Bürsten und/oder Strahlsanden des Feder-Schenkels mit dem zugehörigen Griffelement aufweisen.

Ferner kann das Verfahren einen Schritt einer Gesamtmontage des Instruments umfassen.

Mit anderen Worten kann insbesondere ein medizinisches Instrument bereitgestellt werden, bei dem vorzugsweise zwei Blattfedern oder zwei Federdrähte/Federstahldrähte, je Branche (Griffelement) eine, in Sacklöcher oder in Nuten der Branchen hineingesteckt und/oder verschweißt und/oder gelötet und/oder verklebt sind. Da die Federn außer an ihrer Verbindungsteile (in zumindest der Grundstellung) nirgends an den Branchen anliegen, ist eine gute Reinigung bzw. Reinigbarkeit gewährleistet. Die zweiteilige Feder, welche bei Federkonzepten distal über Federkraft, durch eine Gabel-Nasen-Verbindung oder eine Kugel-Pfannen-Verbindung in Kontakt stehen, hat den Vorteil, dass beim Schließen des medizinischen Instruments, insbesondere in Form einer Zange, die Federkraft nicht linear ansteigt. Die Schließkräfte bleiben hierdurch nahezu konstant.

### Kurzbeschreibung der Figuren

Die vorliegende Offenbarung wird nachfolgend anhand bevorzugter Ausführungsformen unter Bezugnahme auf die begleitenden Figuren näher erläutert. Es zeigen:
- Fig. 1: eine Draufsicht eines medizinischen Instruments einer ersten bevorzugten Ausführungsform, bei der die Federeinheit form- und stoffschlüssig mit den Griffen verbunden ist;
- Fig. 2: eine detaillierte Ansicht einer distalen Feder-Kopplung der zwei Blattfedern der Federeinheit des medizinischen Instruments aus Fig. 1;
- Fig. 3: eine Draufsicht eines medizinischen Instruments einer weiteren, zweiten bevorzugten Ausführungsform, bei der die Federeinheit ein distale Y-t-Verbindung aufweist;
- Fig. 4: eine detaillierte Ansicht einer distalen Verbindung der Federeinheit einer weiteren bevorzugten Ausführungsform eines Instruments in Form einer Kugel-Pfannen-Verbindung;
- Fig. 5: eine Draufsicht auf eine Federeinheit einer weiteren bevorzugten Ausführungsform eines medizinischen Instruments;
- Fig. 6: eine Draufsicht auf ein medizinisches Instrument einer bevorzugten Ausführungsform, in welches die Federeinheit aus Fig. 5 eingesetzt ist;
- Fig. 7: eine detaillierte Ansicht der distalen Feder-Kopplung der Federeinheit aus Fig. 6; und
- Fig. 8: ein Flussdiagramm eines Verfahrens zur Herstellung eines medizinischen Instruments gemäß einer bevorzugten Ausführungsform.

Die Figuren sind schematischer Natur und sollen lediglich dem Verständnis der vorliegenden Offenbarung dienen. Gleiche Elemente sind mit denselben Bezugszeichen versehen. Die Merkmale der verschiedenen Ausführungsformen können untereinander ausgetauscht werden. Jegliche Offenbarung im Zusammenhang mit dem Verfahren gemäß der vorliegenden Offenbarung gilt auch für das medizinische Instrument der vorliegenden Offenbarung, ebenso wie jegliche Offenbarung im Zusammenhang mit dem medizinischen Instrument auch für das Verfahren gemäß der vorliegenden Offenbarung gilt.

### Detaillierte Beschreibung bevorzugter Ausführungsformen

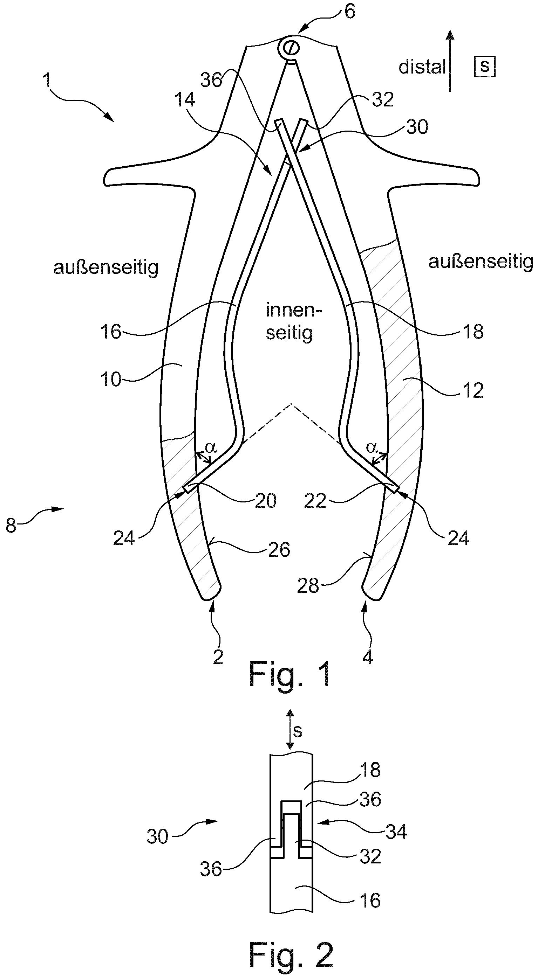

Fig. 1 zeigt in einer Teilansicht ein medizinisches Instrument 1 einer ersten bevorzugten Ausführungsform in einer Grundstellung. Das Instrument 1, in Form eines Handinstruments, ist nach Zangenbauart als Branchenzange ausgebildet. Das Instrument 1 weist dafür zwei Hebel 2, 4 auf, welche miteinander über ein Scharnier 6 schwenkbar verbunden sind. So lassen sich die beiden Hebel 2, 4 in einer Schwenkebene S zueinander, ähnlich einer Schere oder Fasszange, verschwenken. Die proximalen Abschnitte der Hebel 2, 4 (proximal des Scharniers 6 und zum Nutzer weisend) bilden dabei einen Griffabschnitt 8 mit entsprechenden Griffelementen bzw. Griffen 10, 12 aus, die im Wesentlichen symmetrisch zueinander gegenüber einer Instrumentenlängsachse ausgebildet sind. Auch ist der Griffabschnitt 8 symmetrisch zu der Schwenkebene S gestaltet.

Zwischen dem ersten Griff 10 und dem zweiten Griff 12 ist eine zweiteilige Federeinheit 14 in Form einer zweiteiligen Blattfeder vorgesehen, welche einen ersten Feder-Schenkel 16 und einen zweiten Feder-Schenkel 18 der Federeinheit 14 bilden und die an ihren jeweiligen distalen Enden lösbar und verschieblich und schwenkbar zueinander gekoppelt sind.

Die jeweiligen freien Feder-Enden 20, 22 der Feder-Schenkel 16, 18 stehen in eine Vertiefung in Form einer in den jeweiligen Griffen 10, 12 ausgebildeten Nut, die Innenseitig der Griffe 10, 12 an sich gegenüberliegenden innenseitigen Oberflächen 26, 28 in die Griffe 10, 12 hineinstehend ausgebildet ist. Die Vertiefungen 24 sind geradlinig und symmetrisch zueinander gegenüber einer Instrumentenlängsachse ausgebildet und weisen (in der Draufsicht gesehen) leicht schräg nach vorne in distale Richtung. Die Feder-Enden 20, 22 stehen in die Vertiefungen 24 stehen, und bilden so zwischen der distal gelegenen Außenseite der Blattfeder (Feder-Endabschnitt), also der Oberfläche, die nach distal zeigt, und der innenseitigen Oberfläche 26 bzw. 28 einen Auskrag-Winkel α aus. Dies entspricht auch im Wesentlichen einem Winkel zwischen einer Branchenlängsachse bzw. Grifflängsachse entlang des Griffs 10, 12 und dem Feder-Ende 20, 22 bzw. einem sich an das Feder-Ende 20, 22 anschließenden Feder-Endabschnitt bzw. einer Feder-Ende-Längsachse.

Die Feder-Enden 20, 22 sind zudem mit bzw. an den innenseitigen Oberflächen 26, 28 rundum stoffschlüssig verbunden, nämlich verschweißt, so dass ein Spalt zwischen den Vertiefungen 24 und den jeweiligen Feder-Enden zu einer Instrumentenumgebung hin hermetisch verschlossen ist. Auf diese Weise wird sichergestellt, dass einerseits die Federeinheit 14 verliersicher an dem Instrument 1 befestigt bzw. mit diesem verbunden ist und andererseits, dass Spalte und sonstige schwer zugängliche Volumina unterbunden werden, in denen sich Keime festsetzen könnten.

Durch diese spezielle Gestaltung des Instruments 1 mit sowohl formschlüssiger als auch stoffschlüssiger Verbindung von Federeinheit 14 und Griffen 10, 12 und zudem die spezielle Auskragung der Feder-Enden 20, 22 aus den Griffen 10, 12, ermöglicht eine kostengünstige Herstellung des Instruments 1, eine hohe mechanische Belastbarkeit, eine hohe Lebensdauer sowie auch eine außerordentlich gute Reinigbarkeit, insbesondere Sterilisierbarkeit, da insbesondere auch ein ausreichend großer Abstand zwischen den Feder-Schenkeln 16, 18 und den Griffen gewährleistet wird. Da ein Auskrag-Winkel α mit einem Mindestbetrag von ca. 45° gewählt ist, wird ein ausreichend stumpfer Winkel zwischen Feder-Ende 20, 22 und Griff 10, 12 gebildet, der ebenso eine gute Reinigung fördert. Insbesondere stellt der Formschluss mit Vertiefung 24 und zugehörigem Feder-Ende 10, 12 eine ausreichende mechanische Kraft der Federeinheit 14 sicher, so dass das Instrument 1 langlebig ist und eine Funktion auch nach einer sehr hohen Anzahl an Schwenkbewegungen gewährleistet ist. Ein Ausreißen wird verhindert. Die Schweißnaht stellt insbesondere das Verschließen gegenüber der Umgebung sicher und erhöht zusätzlich noch eine mechanische Festigkeit der Anbindung.

Die beiden Blattfedern sind aus dem Werkstoff 1.4021 nach DIN EN 10088 hergestellt, wodurch eine sehr gute mechanische Eigenschaft, gute Polierbarkeit gepaart mit guter chemischer Beständigkeit und Schweißbarkeit bereitgestellt wird.

In Fig. 2 ist detailliert eine distale Feder-Kopplung 30 des Instruments 1 auf Fig. 1 in Form einer distalen Gabel-Nasen-Verbindung gezeigt, in der Richtung von distal nach proximal gesehen. Das freie distale Ende des ersten Feder-Schenkels 16 weist dabei eine Nase 32 auf, die zwischen einer Gabel 34 mit zwei Zacken bzw. Armen 36 bzw. Seitenwänden längsverschieblich und verschwenkbar in Instrumentenlängsachse geführt eingefasst ist. Auf diese Weise kann bei einer Schwenkbewegung des Instruments 1 die distale Feder-Kopplung 30 eine Bewegung mitführen und die Federkraft bleibt über den gesamten Weg einer Verschwenkung konstant. Zudem wird eine Montage vereinfacht. Die beiden distalen Enden sind lösbar miteinander gekoppelt, indem, bedingt durch ihre Vorspannkraft, Nase 32 und Gabel 34 in Eingriff stehen und elastisch gegeneinander gedrückt werden. Es wird gewissermaßen ein federvorgespanntes Scharnier am distalen Ende als Feder-Kopplung 30 geschaffen.

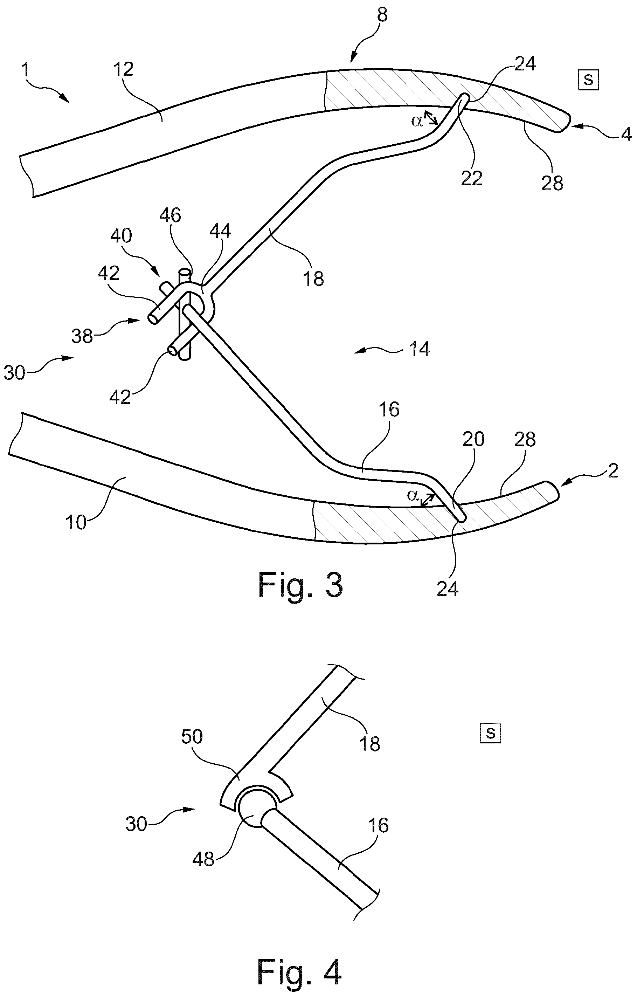

Fig. 3 zeigt ein medizinisches Instrument 1 einer weiteren, zweiten bevorzugten Ausführungsform. Im Unterschied zur ersten Ausführungsform sind die Federeinheit 14 und die zugehörigen Vertiefungen 24 anders gestaltet. Speziell sind die beiden Feder-Schenkel 16, 18 nicht als Blattfedern sondern als S-förmiger Federstahldraht ausgeführt, der in eine Vertiefung 24 einsteht, die als Sackloch ausgeführt ist. Zudem ist die distale Kopplung 30 als Y-t-Verbindung ausgeführt mit einem Y-förmigen distalen Endabschnitt/Y-Abschnitt 38, das verschränkt mit einem t-förmigen distalen Endabschnitt t-Abschnitt 40 lösbar in Kontakt steht und gekoppelt ist. Der Y-Abschnitt 38 weist, wie auch der gesamte zweite Feder-Schenkel 18, in seinen zwei parallel zueinander verlaufenden Y-Armen 42 einen kreisrunden Querschnitt auf. Die Y-Arme 42 laufen die an einer Gabelung 44 geschwungen zusammen. Der t-Abschnitt 40 weist eine senkrecht auf eine Längsachse des ersten Feder-Schenkels 16 stehenden Strebe 46 mit kreisrundem Querschnitt auf, so dass sich gewissermaßen ein t-förmiger oder X-förmiger Abschnitt ausgebildet, der mit dem Y-Abschnitt verschränkt in Kontakt steht. Beide Feder-Schenkel 16, 18 sind gegeneinander vorgespannt. Insbesondere sind in dieser Ausführungsform der Fig. 3 die Streben 46 distal gegenüber den beiden Y-Armen 42 angeordnet, so dass die Streben 46 eine Art Anschlag für den Y-Abschnitt 38 in distaler Richtung bilden. Mit anderen Worten ist der Feder-Schenkel 16 proximal der beiden Streben 46 zwischen den beiden Y-Armen 42 geführt aufgenommen. Aufgrund der Vorspannkraft, insbesondere bei einem Zusammendrücken der beiden Griffe 10, 12, wird der Y-Abschnitt 38 nach distal gedrückt, aber durch die beiden quer zum Feder-Schenkel 16 verlaufenden Streben 46 blockiert. Andererseits wird der t-Abschnitt 40 in der Gabel des Y-Abschnitts 38 gehalten, so dass die beiden Feder-Schenkel 16, 18 lösbar miteinander gekoppelt sind und eine gleichbleibende Federkraft beim gesamten Schwenkbewegungsweg bereitstellen.

Diese Ausführungsform mit einer Y-t-Verbindung ermöglicht eine gute elastische Biegung der beiden Feder-Schenkel 16, 18, die aus Federstahldraht hergestellt und S-förmig (irreversibel) gebogen sind bzw. eine solchen Verlauf entlang ihrer Feder-Schenkel-Längsachse haben, bei gleichzeitiger sicherer (lösbarer) Verbindung an der distalen Feder-Koppelung 30. Diese Ausgestaltung ist insbesondere kostengünstig in der Fertigung.

Fig. 4 zeigt eine detaillierte Ansicht eines distalen Abschnitts der Federeinheit 14, hier die Feder-Kopplung 30 einer weiteren bevorzugten Ausführungsform. Die distale Feder-Kopplung 30 aus Fig. 4 kann in dem Instrument 1 anstelle der Y-t-Verbindung in Fig. 3 eingesetzt werden kann. Im Unterschied zu der Feder-Kopplung 30 der zweiten Ausführungsform aus Fig. 3 weist die Feder-Kopplung der Fig. 4 statt einer Y-t-Verbindung eine Kugel-Pfannen-Verbindung mit einer an dem ersten Feder-Schenkel 16 ausgebildeten distalen Kugel 48 und einer an dem zweiten Feder-Schenkel 18 ausgebildeten Pfanne 50 auf. Konkret liegt die Kugel 48 in einer schalenförmigen Aufnahme der Pfanne 50 federvorgespannt ein und es wird eine (entgegen der Vorspannung der Federeinheit 14) lösbare Verbindung bzw. Kopplung zwischen den distalen Enden geschaffen. Diese spezielle Kopplung bewirkt, dass bei einem Schwenken der Griffe 10, 12 aus einer Grundstellung heraus zunächst die beiden Feder-Schenkel 16, 18 aufgrund der Bewegungsfreiheit nach distal geschoben werden und sich elastisch verformen. Da jedoch durch die Kugel 48 und die Pfanne 50 ein (Kugel-)Gelenk geschaffen wurde, können sich die distalen Enden gegeneinander verschieben und verschwenken, so dass eine Federkraft bei einer Schwenkbewegung wie etwa einer Schließbewegung in einer Schließrichtung gleichmäßig bleibt bzw. eine nahezu konstante Federkraft erreicht wird.

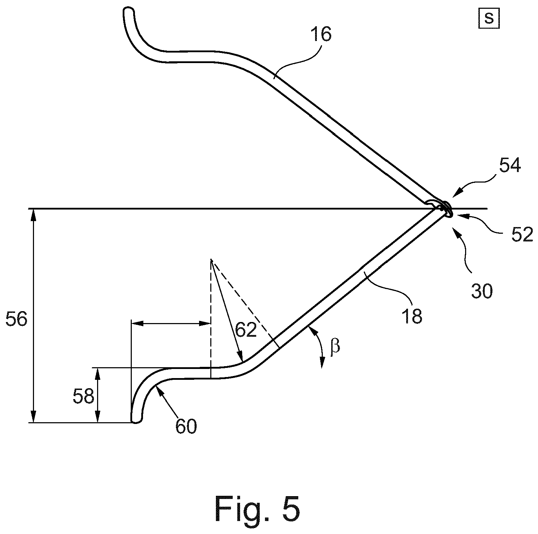

Fig. 5 zeigt eine Federeinheit 14 gemäß einer weiteren bevorzugten Ausführung, welche noch nicht mit den Griffen verbunden ist (und noch nicht vorgespannt ist). Die Feder-Schenkel 16, 18 sind bis auf das distale Ende symmetrisch zueinander ausgebildet. Die proximalen Feder-Enden 20, 22 weisen eine Biegung mit einem ersten proximalen Biegeradius 60 (insbesondere einem Radius R6) auf, der um 90° in einer (in Fig. 5 gesehen am zweiten Feder-Schenkel 18) Rechtskurve verläuft. Nach einem kurzen geradlinigen Abschnitt (insbesondere über eine Länge von 9mm) schließt sich eine (in Fig. 5 gesehen; zweiter Feder-Schenkel 18) Linkskurve um einen Biege-Winkel/Kurven-Winkel β von 45° mit einem größeren distalen Biegeradius 62 (etwa oder genau einem dreifach so großen Biegeradius, insbesondere R17) an. Hiernach schließt sich ein gerader Abschnitt an, an, dessen distaler End-Abschnitt eine leichte Linkskurve (insbesondere um etwa 5°) vollzieht und eine sphärische Rundung als Ende aufweist.

Der erste Feder-Schenkel 16 ist symmetrisch zu dem zweiten Feder-Schenkel 18 S-förmig ausgebildet, weist jedoch an seinem distalen Ende eine pfannenförmige Prägung 52 auf, dessen pfannen-/schalenförmige Aufnahme zum zweiten Feder-Schenkel 18 bzw. zur distalen sphärischen Rundung hin geöffnet ist. In diese Aufnahme ist das sphärische distale Ende des zweiten Feder-Schenkels 18 eingelegt bzw. eingesetzt. Eine maximale Abmessung 56 in Schwenkebene S in Richtung senkrecht zur Instrumentenlängsachse beträgt im nicht vorgespannten Zustand insbesondere etwa das 1,3 fache einer maximalen Abmessung in Schwenkebene S in Richtung Instrumentenlängsachse. Eine Abmessung 58 einer Auskragung des Feder-Endes 20 in der Schwenkebene S in Richtung senkrecht zu einer Instrumentenlängsachse beträgt in etwa 20% der maximalen Abmessung 56. Insbesondere beträgt eine Abmessung 58 der Auskragung 11mm. Insbesondere weist das Feder-Ende 20, 22 einen geradlinigen Abschnitt auf, der sich etwa über ein Drittel oder die Hälfte der Abmessung 58 der Auskragung erstreckt, insbesondere über 5mm.

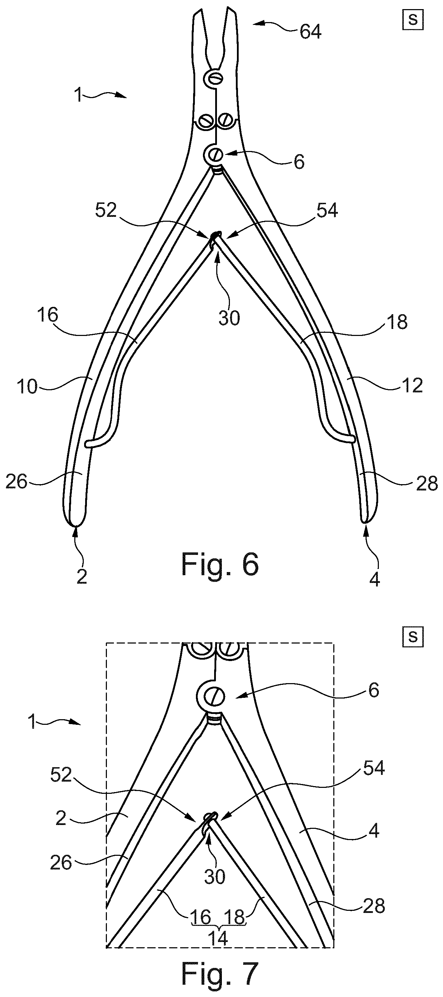

Fig. 6 und Fig. 7 zeigen in einer Draufsicht sowie einer detaillierten Draufsicht im Bereich der distalen Feder-Kopplung 30 ein medizinisches Instrument 1 gemäß einer weiteren bevorzugten Ausführungsform. Das medizinische Instrument 1 ist als mehrgelenkige Branchenzange ausgebildet und weist einen distalen Wirkabschnitt 64 in Form einer Zange auf. Die Federeinheit 14 aus Fig. 5 ist in diesem Instrument eingesetzt. Konkret stehen die beiden Feder-Enden 20, 22 an den innenseitigen Oberflächen 26, 28 in Vertiefungen (nicht dargestellt) der Griffe 10, 12 hinein.

Ein Auskragwinkel α ist größer als in der ersten Ausführungsform und beträgt ca. 80°. An das Feder-Ende 20, 22 schließt sich jeweils der proximale Biegeradius 60 an. In Folge ist innenseitig der Griffe 10, 12 ein definiert gestalteter Spalt vorgesehen, der eine hohe Mindestgröße hat und aufgrund der Rundungen sowohl bedingt durch den kreisförmigen Querschnitt des S-förmig gebogenen Federstahldrahts und dem Biegeradius 60 eine gute Reinigbarkeit sicherstellt. Zudem lässt sich das Instrument 1 einfach und kostengünstig herstellen.

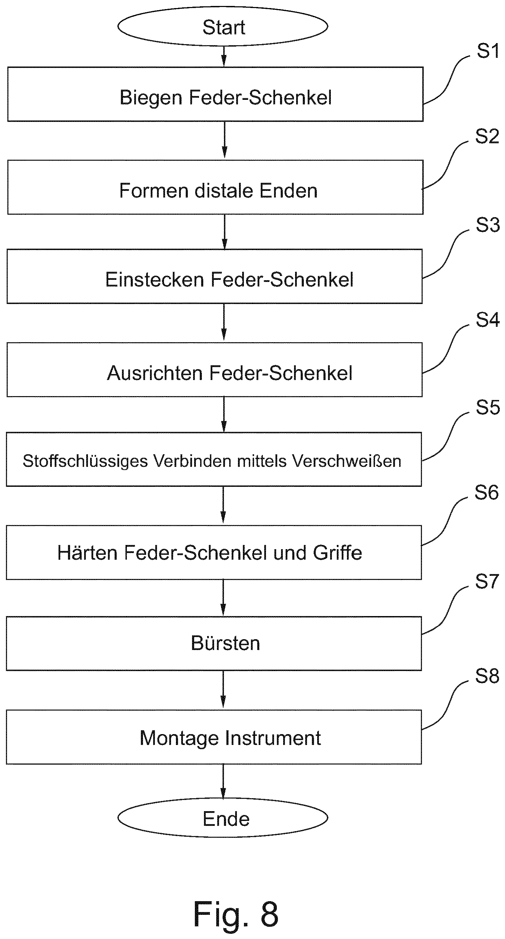

Fig. 8 zeigt ein Flussdiagramm eines Verfahrens zur Herstellung eines Instruments 1 gemäß einer bevorzugten Ausführungsform. In dieser Ausführungsform wird durch das Verfahren ein Instrument 1 gemäß der vorliegenden Offenbarung hergestellt.

In einem ersten Schritt S1 werden sowohl ein erster also und ein zweiter Feder-Schenkel 16, 18 in die richtige Form gebogen. Insbesondere ist der Feder-Schenkel 16, 18 in Form eines Federstahldrahts oder einer Blattfeder ausgebildet und wird S-förmig (nach einer Skizze) gebogen.

In einem Schritt S2 werden die distalen Abschnitte der beiden Feder-Schenkel 16, 18 geformt, die später distal miteinander gekoppelt werden. Konkret wird bei dem ersten Feder-Schenkel 16 eine Prägung des freien Endes vorgenommen, derart, dass eine flache löffelförmige oder pfannenförmige Struktur/Pfanne 52 herausgebildet wird, die eine schalenförmige Aufnahme ausformt. Das andere distale Ende des zweiten Feder-Schenkels 18 wird distal angeschliffen um eine kugelförmige/sphärische Rundung 54 zu erhalten.

Die Reihenfolge der Schritte S1 und S2 ist nicht entscheidend, so dass diese beiden Schritte auch in umgedrehter Reihenfolge durchgeführt werden können.

Hiernach erfolgt der Schritt S3 eines Einsteckens der beiden Feder-Schenkel 16, 18 in jeweils eine in den Griffen 10, 12 ausgebildete Vertiefung 24 in Form eines Sacklochs, so dass eine formschlüssige Verbindung zwischen den Feder-Schenkeln 16, 18 und den zugehörigen Griffen 10, 12 geschaffen wird.

Anschließend werden die beiden Feder-Schenkel 16, 18 in einem Schritt S4 gegenüber den zugehörigen Griffen 10, 12 ausgerichtet. Insbesondere kann auch bereits zu diesem Zeitpunkt die distale kugelförmige Rundung lose in die komplementäre Pfanne 52 eingelegt und die beiden Feder-Schenkeln 16, 18 gegeneinander vorgespannt werden, um ein korrekte Ausrichtung zu überprüfen.

In dieser ausgerichteten Stellung werden dann die Federschenkel in Schritt S5 stoffschlüssig miteinander verbunden. Vorliegend werden die Feder-Schenkel mit dem zugehörigen Griffelement, insbesondere mit Zusatz, verschweißt. Konkret wird jeweils eine um die Feder-Enden 20, 22 bzw. an den Endabschnitten umlaufende Schweißnaht an den Griffen 10, 12 gebildet, so dass ein sich ausbildender Spalt zwischen den Feder-Enden 20, 22 und der Vertiefung 24 hermetisch versiegelt wird.

Nach dem Schritt S5 des stoffschlüssigen Verbindens erfolgt der Schritt S6 des Härtens der Feder-Schenkel 16,18 mit dem zugehörigen Griffelement 10, 12. Vorliegend wird insbesondere das gesamte Instrument 1 bzw. werden alle Komponenten gehärtet.

In Schritt S7 werden die Griffe 10, 12 und die Federeinheit 14 gebürstet und gesandstrahlt, um insbesondere an der Verbindungsstelle von Griff 10, 12 und Feder-Schenkel 16, 18 eine glatte Oberfläche zu schaffen.

Schließlich folgt in Schritt S8 die Gesamtmontage des Instruments 1.

### Bezugszeichenliste

- 1: Medizinisches Instrument
- 2: Erster Hebel
- 4: Zweiter Hebel
- 6: Scharnier
- 8: Griffabschnitt
- 10: Erstes Griffelement
- 12: Zweites Griffelement
- 14: Federeinheit
- 16: Erster Feder-Schenkel
- 18: Zweiter Feder-Schenkel
- 20: Erstes Feder-Ende
- 22: Zweites Feder-Ende
- 24: Vertiefung
- 26: Erste innenseitige Oberfläche
- 28: Zweite innenseitige Oberfläche
- 30: Distale Feder-Kopplung
- 32: Nase
- 34: Gabel
- 36: Gabel-Arm
- 38: Y-Abschnitt
- 40: t-Abschnitt
- 42: Y-Arm
- 44: Y-Gabelung
- 46: Strebe
- 48: Kugel
- 50: Pfanne
- 52: Prägung
- 54: Gebogenes Ende
- 56: Abmessung
- 58: Abmessung Auskragung
- 60: (Erster) proximaler Biegeradius
- 62: (Zweiter) distaler Biegeradius

- S: Schwenkebene
- α: Auskrag-Winkel
- β: Kurven-Winkel

- S1: Schritt Biegen Feder-Schenkel
- S2: Schritt Formen distales Ende
- S3: Schritt Einstecken Feder-Schenkel
- S4: Schritt Ausrichten Feder-Schenkel gegenüber Griff
- S5: Schritt Stoffschlüssiges Verbinden
- S6: Schritt Härten
- S7: Schritt Bürsten
- S8: Schritt Montage

## Patentansprüche

1. Medizinisches, insbesondere chirurgisches, Instrument (1), mit zwei Griffelementen (10, 12), die Metall als Material aufweisen, insbesondere insgesamt aus Metall als Material hergestellt sind, und die über ein Lager, insbesondere über ein Gelenk, schwenkbar zueinander gelagert sind, und einer Federeinheit (14), welche zwei Feder-Enden (20, 22) aufweist, die jeweils mit einem der beiden Griffelementen (10, 12) verbunden sind, so dass bei Verschwenken zumindest eines der beiden Griffelemente (10, 12) aus einer Grundstellung ein Zurückschwenken in die Grundstellung mittels der Federeinheit (14) durchführbar ist, **dadurch gekennzeichnet, dass**
die Federeinheit (14) sich von zumindest einem der beiden Feder-Enden (20,22) aus, vorzugsweise von beiden Feder-Enden (20, 22) aus, hin zu dem Lager, insbesondere Gelenk, erstreckt und dafür angepasst ist bei Verschwenken der Griffelemente (10, 12) eine im Wesentlichen gleichbleibende Federkraft bereitzustellen, insbesondere zumindest zweiteilig ausgebildet ist und einen ersten Feder-Schenkel (16) und einen zweiten Feder-Schenkel (18) aufweist, und
zumindest eines der beiden Feder-Enden (20, 22) in eine in dem zugehörigen Griffelement (10, 12) ausgebildete Vertiefung (24) hineinsteht und mit dem zugehörigen Griffelement (10, 12) stoffschlüssig verbunden ist, insbesondere derart, dass ein Spalt zwischen Vertiefung (24) und Feder-Ende (20, 22) zu einer Instrumentenumgebung hin hermetisch verschlossen ist.

2. Medizinisches Instrument (1) nach Anspruch 1, **dadurch**
**gekennzeichnet, dass** das zumindest eine Feder-Ende (20, 22) und das zugehörige Griffelement (10, 12) miteinander Verschweißt und/oder Verlötet, insbesondere mit einem Zusatz Hartgelötet, und/oder Verklebt sind, insbesondere an einer Kontaktregion zwischen einer Außenfläche des Feder-Endes (20, 22) und einer innenseitigen Oberfläche (26, 28) des zugehörigen Griffelements (10, 12), die dem anderen Griffelement (10, 12) gegenüberliegt.

3. Medizinisches Instrument (1) nach Anspruch 1 oder 2, **dadurch**
**gekennzeichnet, dass** zumindest ein Feder-Ende (20, 22), insbesondere zumindest einer der beiden Feder-Schenkel (16, 18), einen Auskrag-Winkel (α) zwischen dem, insbesondere einstehenden, Feder-Ende (20, 22) und dem zugehörigen Griffelement (10, 12) von mindestens 30°, bevorzugt von mindestens 45°, ganz bevorzugt von mindestens 70° und insbesondere von genau 90° aufweist.

4. Medizinisches Instrument (1) nach einem der vorstehenden Ansprüche, **dadurch gekennzeichnet, dass** zumindest ein Feder-Schenkel (16, 18) als Blattfeder oder als Federstahldraht ausgebildet ist und insbesondere als Material den Werkstoff X20CR13 oder nach DIN EN 10088 den Werkstoff 1.4021 aufweist, insbesondere insgesamt aus diesem hergestellt ist.

5. Medizinisches Instrument (1) nach Anspruch 4, **dadurch**
**gekennzeichnet, dass** die Federeinheit (14) zweiteilig in Form von zwei Blattfedern oder zwei Federstahldrähten ausgebildet ist, die über eine distale Feder-kopplung (30), insbesondere über eine distale Gabel-Nasen-Verbindung (32, 34) oder eine distale Kugel-Pfannen-Verbindung (48, 50) oder eine distale Y-t-Verbindung (38, 40) miteinander lösbar gekoppelt sind.

6. Medizinisches Instrument (1) nach einem der vorstehenden Ansprüche, **dadurch gekennzeichnet, dass** zumindest einer der beiden Feder-Schenkel (16, 18), insbesondere beide, S-förmig ausgebildet ist, mit insbesondere einem ersten proximalen Biegeradius (60) und/oder einem gegenläufigen, insbesondere größeren, distalen Biegeradius (62), wobei vorzugsweise ein Biege-Winkel zwischen den tangentialen Abschnitten des proximalen Biegeradius 90° und/oder des distalen Biegeradius 45° beträgt.

7. Medizinisches Instrument (1) nach einem der vorstehenden Ansprüche, **dadurch gekennzeichnet, dass** die Vertiefung (24) ein Sackloch oder eine Nut oder ein Schlitz, insbesondere ein Durchgangsschlitz, ist, und insbesondere eine Tiefe von mindestens 2mm und/oder maximal 6mm aufweist.

8. Medizinisches Instrument (1) nach einem der vorstehenden Ansprüche, **dadurch gekennzeichnet, dass** die Federeinheit (14) gegenüber den Griffelementen (10, 12) derart angepasst ist, dass die Federeinheit (14), insbesondere der erste Federschenkel (16) und/oder der zweite Feder-Schenkel (18), in zumindest der Grundstellung, insbesondere in allen Stellungen einer Verschwenkung der Griffelemente (10, 12) zueinander, nur an dem Feder-Ende (20, 22) als Verbindungsstelle mit den zugehörigen Griffelementen (10, 12) in Kontakt kommt.

9. Verfahren zur Herstellung eines medizinischen Instruments mit einer Federeinheit, insbesondere eines Instruments (1) nach einem der Ansprüche 1 bis 8, **gekennzeichnet durch** die Schritte, insbesondere in dieser Reihenfolge:
- Biegen (S1) zumindest eines Feder-Schenkels (16, 18), insbesondere einer Blattfeder oder eines Federstahldrahts;
- Formen (S2), insbesondere Prägen und/oder Anschleifen, eines distalen Abschnitts oder Endes des Feder-Schenkels (16, 18), vorzugsweise Anschleifen einer distalen Rundung;
- Einstecken (S3) des zumindest einen Feder-Schenkels (16, 18) in eine in einem Griffelement (10, 12) ausgebildete Vertiefung (24), insbesondere in Form eines Sacklochs oder einer Nut;
- Vorzugsweise Ausrichten (S4) des Feder-Schenkels (16, 18) gegenüber dem zugehörigen Griffelement (10, 12), insbesondere mit einer Erstreckung des eingesteckten Endes des Feder-Schenkels (16, 18) hin zu einem Lager, vorzugsweise Gelenk, des medizinischen Instruments, über welches die Griffelemente schwenkbar zueinander gelagert sind;
- Stoffschlüssiges Verbinden (S5), insbesondere Verschweißen und/oder Löten des Feder-Schenkels (16, 18) mit dem zugehörigen Griffelement (10, 12).

10. Verfahren nach Anspruch 9, **dadurch gekennzeichnet, dass** das Verfahren nach dem Schritt des stoffschlüssigen Verbindens (S5) den Schritt Härten (S6) des Feder-Schenkels mit dem zugehörigen Griffelement aufweist und/oder den Schritt Bürsten (S7) und/oder Sandstrahlen des Feder-Schenkels (16, 18) mit dem zugehörigen Griffelement (10, 12) aufweist.

## Claims

1. A medical, in particular surgical, instrument (1), having two gripping elements (10, 12) which have metal as material, are particularly entirely made from metal as material, and which are pivotably mounted relative to each other via a bearing, in particular via a joint, and a spring unit (14), which has two spring ends (20, 22) which are each connected to one of the two gripping elements (10, 12), so that when at least one of the two gripping elements (10, 12) pivots out of a base position, pivoting back into the base position can be carried out via the spring unit (14), **characterized in that**
the spring unit (14) extends from at least one of the two spring ends (20, 22), preferably from both spring ends (20, 22), toward the bearing, in particular joint and is adapted to provide a substantially constant spring force when the gripping elements (10, 12) pivot, is in particular formed of at least two parts and has a first spring leg (16) and a second spring leg (18), and
at least one of the two spring ends (20, 22) protrudes into an indentation (24) formed in the associated gripping element (10, 12) and/or is firmly bonded to the associated gripping element (10, 12), in particular so that a gap between indentation (24) and spring end (20, 22) is hermetically sealed toward an instrument environment.

2. The medical instrument (1) according to claim 1, **characterized in that** the at least one spring end (20, 22) and the associated gripping element (10, 12) are welded and/or soldered together, in particular brazed with an additive, and/or glued, in particular at a contact region between an outer surface of the spring end (20, 22) and an internal surface (26, 28) of the associated gripping element (10, 12), which is opposite the other gripping element (10, 12).

3. The medical instrument (1) according to claim 1 or 2, **characterized in that** at least one spring end (20, 22), in particular at least one of the two spring legs (16, 18), has a cantilever angle (α) between the in particular protruding spring end (20, 22) and the associated gripping element (10, 12) of at least 30°, preferably of at least 45°, most preferably of at least 70°, and in particular of exactly 90°.

4. The medical instrument (1) according to any of the preceding claims,
**characterized in that** at least one spring leg (16, 18) is configured as a leaf spring or spring steel wire and, in particular, has the material X20CR13 or, according to DIN EN 10088, the material 1.4021 as the material, in particular is made entirely of this material.

5. The medical instrument (1) according to claim 4, **characterized in that** the spring unit (14) is configured in two parts in the form of two leaf springs or two spring steel wires, which are detachably coupled to each other via a distal spring coupling (30), in particular via a distal fork-nose connection (32, 34) or a distal ball pan connection (48, 50) or a distal Y-t connection (38, 40).

6. The medical instrument (1) according to any of the preceding claims,
**characterized in that** at least one of the two spring legs (16, 18), in particular both, is S-shaped with in particular a first proximal bending radius (60) and/or an opposing, in particular larger, distal bending radius (62), a bending angle between the tangential portions of the proximal bending radius preferably being 90° and/or of the distal bending radius being 45°.

7. The medical instrument (1) according to any of the preceding claims, **characterized in that** the indentation (24) is a blind hole or a groove or a slit, in particular a passage slit, and in particular has a depth of at least 2 mm and/or a maximum of 6 mm.

8. The medical instrument (1) according to any of the preceding claims,
**characterized in that** the spring unit (14) is adapted with respect to the gripping elements (10, 12) in such a way that the spring unit (14), in particular the first spring leg (16) and/or the second spring leg (18), in at least the base position, in particular in all positions in which gripping elements (10, 12) are pivoted to each other, only comes into contact with the associated gripping elements (10, 12) at the spring end (20, 22) as the connection point.

9. A method for manufacturing a medical instrument with a spring unit, in particular an instrument (1) according to any of claims 1 to 8, **characterized by** the steps, in particular in this order:
- bending (S1) at least one spring leg (16, 18), in particular of a leaf spring or a spring steel wire;
- forming (S2), in particular imprinting and/or grinding, a distal portion or end of the spring leg (16, 18), preferably grinding a distal rounding;
- inserting (S3) the at least one spring leg (16, 18) into an indentation (24) formed in a gripping element (10, 12), in particular in the form of a blind hole or a groove;
- preferably orienting (S4) the spring leg (16, 18) relative to the associated gripping element (10, 12), in particular with an extension of the inserted end of the spring leg (16, 18) toward a bearing, preferably joint, of the medical instrument, via which the gripping elements are pivotably mounted relative to each other;
- firmly bonded connecting (S5), in particular welding and/or soldering, the spring leg (16, 18) to the associated gripping element (10, 12).

10. The method according to claim 9, **characterized in that,** after the step of firmly bonded connecting (S5), the method comprises the step of tempering (S6) the spring leg with the associated gripping element and/or comprises the step of brushing (S7) and/or sandblasting the spring leg (16, 18) with the associated gripping element (10, 12).

## Revendications

1. Instrument médical, en particulier chirurgical (1), avec deux éléments de préhension (10, 12), qui présentent du métal en guise de matériau, en particulier sont fabriqués dans l'ensemble en métal en guise de matériau, et qui sont montés par le biais d'un palier, en particulier par le biais d'une articulation, de manière à pouvoir pivoter l'un par rapport à l'autre, et une unité de ressort (14) qui présente deux extrémités de ressort (20, 22) qui sont reliées respectivement à l'un des deux éléments de préhension (10, 12) de sorte que lors du pivotement d'au moins l'un des deux éléments de préhension (10, 12) à partir d'une position initiale, un pivotement retour dans la position initiale puisse être réalisé au moyen de l'unité de ressort (14), **caractérisé en ce que**
l'unité de ressort (14) s'étend depuis au moins l'une des deux extrémités de ressort (20, 22), de préférence depuis les deux extrémités de ressort (20, 22), vers le palier, en particulier l'articulation, et est adaptée pour fournir une force de ressort sensiblement constante lors du pivotement des éléments de préhension (10, 12), en particulier est conçue au moins en deux parties et présente une première branche à ressort (16) et une seconde branche à ressort (18), et
au moins l'une des deux extrémités de ressort (20, 22) pénètre dans une cavité (24) formée dans l'élément de préhension (10, 12) afférent et est reliée par accouplement de matière à l'élément de préhension (10, 12) afférent, en particulier de telle manière qu'une fente entre la cavité (24) et l'extrémité de ressort (20, 22) soit fermée hermétiquement par rapport à un environnement d'instrument.

2. Instrument médical (1) selon la revendication 1, **caractérisé en ce que** la au moins une extrémité de ressort (20, 22) et l'élément de préhension (10, 12) afférent sont soudés et/ou brasés l'un à l'autre, en particulier brasés avec un additif et/ou collés, en particulier au niveau d'une région de contact entre une surface extérieure de l'extrémité de ressort (20, 22) et une surface côté intérieur (26, 28) de l'élément de préhension (10, 12) afférent qui fait face à l'autre élément de préhension (10, 12).

3. Instrument médical (1) selon la revendication 1 ou 2, **caractérisé en ce qu'** au moins une extrémité de ressort (20, 22), en particulier au moins d'une des deux branches à ressort (16, 18) présente un angle en saillie (α) entre l'extrémité de ressort (20, 22) en particulier en place, et l'élément de préhension (10, 12) afférent d'au moins 30°, de préférence d'au moins 45°, tout préférentiellement d'au moins 70° et en particulier précisément de 90°.

4. Instrument médical (1) selon l'une quelconque des revendications précédentes, **caractérisé en ce qu'** au moins une branche à ressort (16, 18) est formée comme ressort à lames ou comme fil d'acier à ressort et en particulier présente en guise de matériau le matériau X20CR13 ou selon DIN EN 10088 le matériau 1.4021, en particulier est fabriqué dans l'ensemble en celui-ci.

5. Instrument médical (1) selon la revendication 4, **caractérisé en ce que** l'unité de ressort (14) est conçue en deux parties sous la forme de deux ressorts à lame ou deux fils d'acier à ressort qui sont couplés l'un à l'autre de manière amovible par l'intermédiaire d'un couplage de ressort (30) distal, en particulier d'une liaison fourche-nez (32, 34) distale ou d'une liaison bille-cannelure (48, 50) distale ou d'une liaison Y-t distale.

6. Instrument médical (1) selon l'une quelconque des revendications précédentes, **caractérisé en ce qu'** au moins l'une des deux branches à ressort (16, 18), en particulier les deux, est formée en S, avec en particulier un premier rayon de flexion (60) proximal et/ou un rayon de flexion (62) distal contraire, en particulier plus grand, dans lequel un angle de flexion entre les sections tangentielles du rayon de flexion proximal s'élève de préférence à 90° et/ou du rayon de flexion distal s'élève de préférence à 45°.

7. Instrument médical (1) selon l'une quelconque des revendications précédentes, **caractérisé en ce que** la cavité (24) est un trou borgne ou une rainure ou une fente, en particulier une fente traversante, et présente en particulier une profondeur d'au moins 2 mm et/ou au maximum de 6 mm.

8. Instrument médical (1) selon l'une quelconque des revendications précédentes, **caractérisé en ce**que l'unité de ressort (14) est adaptée par rapport aux éléments de préhension (10, 12) de telle manière que l'unité de ressort (14), en particulier la première branche à ressort (16) et/ou la seconde branche à ressort (18), vienne en contact dans au moins la position initiale, en particulier dans toutes les positions d'un pivotement des éléments de préhension (10, 12) l'un par rapport à l'autre, uniquement au niveau de l'extrémité de ressort (20, 22) comme point de liaison avec les éléments de préhension (10, 12) afférents.

9. Procédé de fabrication d'un instrument médical avec une unité de ressort, en particulier d'un instrument (1) selon l'une quelconque des revendications 1 à 8, **caractérisé par** les étapes suivantes, en particulier dans cet ordre :
- la flexion (S1) au moins d'une branche à ressort (16, 18), en particulier un ressort à lames ou un fil d'acier à ressort ;
- la formation (S2), en particulier l'estampage et/ou le ponçage, d'une section distale ou extrémité de la branche à ressort (16, 18), de préférence le ponçage d'un arrondi distal ;
- l'insertion (S3) d'au moins une branche à ressort (16, 18) dans une cavité (24) formée dans un élément de préhension (10, 12), en particulier sous la forme d'un trou borgne ou d'une rainure ;
- de préférence l'orientation (S4) de la branche à ressort (16, 18) par rapport à l'élément de préhension (10, 12) afférent, en particulier avec une étendue de l'extrémité insérée de la branche à ressort (16, 18) vers un palier, de préférence une articulation, de l'instrument médical, par le biais duquel les éléments de préhension sont logés de manière pivotante l'un par rapport à l'autre ;
- la liaison par accouplement de matière (S5), en particulier le soudage et/ou le brasage de la branche à ressort (16, 18) avec l'élément de préhension (10, 12) afférent.

10. Procédé selon la revendication 9, **caractérisé en ce que** le procédé présente, après l'étape de liaison par accouplement de matière (S5), l'étape de durcissement (S6) de la branche à ressort avec l'élément de préhension afférent et/ou l'étape de brossage (S7) et/ou de sablage de la branche à ressort (16, 18) avec l'élément de préhension (10, 12) afférent.
